(19) Europäisches Patentamt
European Patent Office
Office européen des brevets

(11) EP 0 890 176 B1

(12) # FASCICULE DE BREVET EUROPEEN

(45) Date de publication et mention de la délivrance du brevet:
**20.06.2001 Bulletin 2001/25**

(21) Numéro de dépôt: **97951052.6**

(22) Date de dépôt: **30.12.1997**

(51) Int Cl.[7]: **H01B 1/12**, G02F 1/15

(86) Numéro de dépôt international:
**PCT/CA97/01012**

(87) Numéro de publication internationale:
**WO 98/29877 (09.07.1998 Gazette 1998/27)**

(54) **CONDUCTEURS PROTONIQUES SOUS FORME LIQUIDE**

FLÜSSIGER PROTONENLEITER

PROTON CONDUCTOR IN LIQUID FORM

(84) Etats contractants désignés:
**DE FR GB IT**

(30) Priorité: **30.12.1996 CA 2194127**
**05.03.1997 CA 2199231**

(43) Date de publication de la demande:
**13.01.1999 Bulletin 1999/02**

(73) Titulaires:
- **HYDRO-QUEBEC**
  **Montréal Québec H2Z 1A4 (CA)**
- **CENTRE NATIONAL DE LA RECHERCHE SCIENTIFIQUE (CNRS)**
  **75016 Paris (FR)**

(72) Inventeurs:
- **ARMAND, Michel**
  **Montréal, Québec H3T 1N2 (CA)**
- **MICHOT, Christophe**
  **F-38000 Grenoble (FR)**
- **KAPFER, Bruno**
  **Montréal, Québec H2Z 1A4 (CA)**

(74) Mandataire: **Sueur, Yvette et al**
**Cabinet SUEUR & L'HELGOUALCH,**
**109, boulevard Haussmann**
**75008 Paris (FR)**

(56) Documents cités:
**WO-A-96/13872**
**WO-A-98/07164**
**DE-A- 19 632 285**
**US-A- 4 664 757**
**US-A- 4 664 761**
**US-A- 5 283 310**

- **KREUER, KLAUS-DIETER: "Proton Conductivity: Materials and Applications" CHEM. MATER. (1996), 8(3), 610-41 CODEN: CMATEX;ISSN: 0897-4756, mars 1996, XP002061708**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Printed by Jouve, 75001 PARIS (FR)

EP 0 890 176 B1

## Description

### Domaine Technique

La présente invention est relative à de nouveaux conducteurs protoniques sous forme liquide et à leur utilisation comme électrolytes liquides, gels ou polymères dans divers systèmes électrochimiques.

### Technique Antérieure

Les conducteurs protoniques les plus usuels sont obtenus par le dopage d'eau par un acide tel que HCl ou $H_2SO_4$, ou une base telle que KOH ou $NH_3$. Ainsi, les solutions d'acide sulfurique dans l'eau présentent une conductivité protonique élevée supérieure à $10^{-2}$ $S.cm^{-1}$ et les solutions d'hydroxyde de potassium également très conductrices sont largement utilisées comme électrolyte dans les batteries Nickel-Cadmium.

D'une façon générale, les conducteurs protoniques nécessitant la présence d'eau pour leur fonctionnement ont un domaine d'utilisation limité en température du fait de l'évaporation de cette eau et un domaine de stabilité rédox limité à celui de l'eau. La présence d'eau induit également couramment des phénomènes de corrosion au sein des systèmes utilisant ces électrolytes.

Pour pallier ces problèmes rédhibitoires vis à vis de certaines applications, de nombreux travaux ont portés sur l'étude de conducteurs protoniques anhydres. Parmi les différentes classes de matériaux issues de ces recherches, on a plus particulièrement obtenu des conducteurs protoniques anhydres en substituant le solvant aqueux par des polymères solvatants non-hydroxyliques tels le poly(oxyde d'éthylène), la polyvinylpyrrolidone, la polyéthylèneimine ou le poly(aminopropylsiloxane).

En dopant ces polymères par des acides ou des bases, on a obtenu des conducteurs protoniques anhydres. Par exemple, la dissolution d'acide orthophosphorique $H_3PO_4$ dans du poly(oxyde d'éthylène) (POE) permet d'obtenir un conducteur protonique acide, la dissolution de sulfamide $H_2NSO_2NH_2$ dans ce même polymère permettant d'obtenir un conducteur protonique basique.

Ces électrolytes sont utiles pour la réalisation de systèmes électrochimiques, en particulier les systèmes de modulation de la lumière, mais ils présentent néanmoins des inconvénients majeurs. Les conducteurs protoniques basés sur la dissolution de $H_3PO_4$ dans du POE sont corrosifs du fait de la forte acidité du milieu ($pK_a \approx 0$). A l'inverse, la forte basicité ($pK_a \approx$ 11-12) des conducteurs protoniques basés sur la dissolution de sulfamides dans du POE limitent leurs utilisations, en effet en présence de nombreux matériaux d'électrodes contenant des espèces métalliques, il se forme une couche de passivation peu conductrice de cations métalliques complexés avec la sulfamide. D'autre part, ces électrolytes sont peu conducteurs à basse température.

DE-196 32 285 décrit des conducteurs protoniques obtenus par mélange d'une base azotée non aqueuse et d'un acide, les proportions respectives des constituants variant de 1% à 99%. Une membrane à conduction protonique est ainsi obtenue par mise en contact de pyrazole, respectivement d'imidazole, avec une membrane de polyéthercétone sulfonée à une température de l'ordre de 80-90°C pour le pyrazole et de 100-110°C pour l'imidazole. La conductivité des membranes est de 0,02, respectivement de 0,01 S/cm à température élevée (200°C).

### Exposé de l'Invention

Afin de pallier les inconvénients ci-dessus, les inventeurs ont découvert que, de façon surprenante, des mélanges binaires de certaines bases azotées appartenant à la famille des azoles et de sels d'addition de ces bases azotées avec des acides appropriés formaient des conducteurs protoniques dont le point de fusion est inférieure à la température ambiante, d'une faible tension de vapeur, dont la conductivité se rapproche de celle obtenue en milieux aqueux, et dont la conductivité à basse température est supérieure à la conductivité à basse température des conducteurs protoniques de l'art antérieur.

La présente invention a donc pour objet un conducteur protonique sous forme liquide, caractérisé par le fait qu'il est constitué d'un mélange des composants (a) et (b) suivants:

a) un sel d'addition d'une base azotée avec un acide, répondant à la formule:

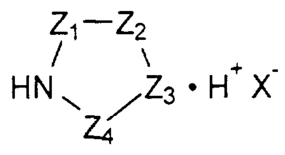

dans laquelle:

$Z_1$, $Z_2$, $Z_3$ et $Z_4$, identiques ou différents, représentent un groupement -N= ou -C($Y_i$)= dans lequel $Y_i$ désigne un atome d'hydrogène, un radical alkyle linéaire ou ramifié ayant de 1 à 20 atomes de carbone, un radical fluoroalkyle ayant de 1 à 20 atomes de carbone, ou un radical oxoalkyle ou azaalkyle ayant de 1 à 20 atomes de carbone, sous réserve qu'au moins un et au plus deux des groupements $Z_1$, $Z_2$, $Z_3$ et $Z_4$ représente -N=, deux atomes de carbone adjacents étant éventuellement hydrogénés et la base azotée faisant éventuellement partie d'une trame polymérique, et

$X^-$ représente un anion dérivé d'un acide choisi dans le groupe constitué par les acides sulfoniques de formule $R_FSO_3H$, les sulfonimides de formule $(R_FSO_2)$ $(R'_FSO_2)NH$ et les méthylures de formule $(R_FSO_2)$ $(R'_FSO_2)$ $CH_2$ ou $(R_FSO_2)$ $(R'_FSO_2)$ $(R''_FSO_2)$ CH, dans lesquelles $R_F$, $R'_F$ ou $R''_F$ représentent indépendamment un radical $F(CF_2)_n$-, n étant compris entre O et 6, l'acide faisant éventuellement partie d'une trame polymérique; et

b) une base azotée répondant à la formule:

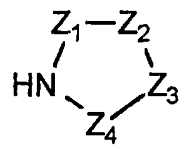

dans laquelle $Z_1$, $Z_2$, $Z_3$ et $Z_4$ ont les significations précitées, la base azotée faisant éventuellement partie d'une trame polymérique;

les composants (a) et (b) étant présents dans des proportions telles à former une composition ayant un point de fusion inférieure à 25°C.

L'invention a également pour objet un électrolyte liquide caractérisé par le fait qu'il est constitué d'un conducteur protonique tel que défini ci-dessus.

L'invention vise également un électrolyte polymère caractérisé par le fait qu'il comprend un conducteur protonique tel que défini ci-dessus dissout dans un polymère omportant au moins un groupement polaire.

De préférence, la composition formée par le mélange des composants (a) et (b) est ,sensiblement eutectique.

À titre d'exemples de bases azotées, on peut mentionner les azoles de formules:

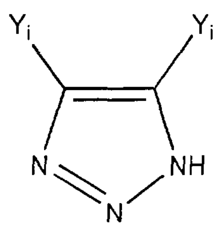

1,2,3-triazole

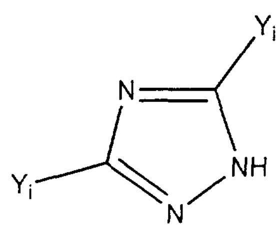

1,3,4-triazole

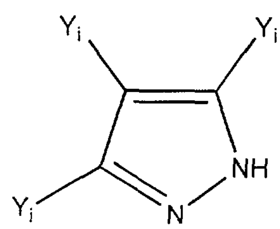

pyrazole

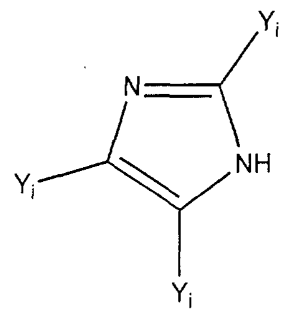

imidazole

dans lesquelles $Y_i$ a la signification précitée.

On considère également les azoles partiellement hydrogénés tels les imidazolines de formule:

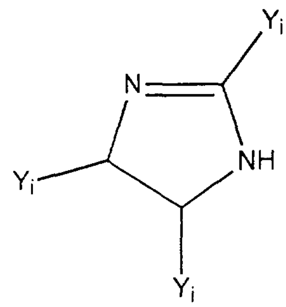

dans laquelle $Y_i$ a la signification précitée.

Le choix de la base azotée permet de contrôler le pH des conducteurs protoniques selon l'invention. Ainsi, les mélanges binaires triazole/triazolium ont un $pK_a$ de l'ordre de 2, les mélanges binaires imidazole/imidazolium ont un $pK_a$ de l'ordre de 7, et les mélanges binaires imadazoline/imidazolinium ont un $pK_a$ de l'ordre de 10.

À titre d'exemples d'acides, on peut mentionner l'acide triflique, la bisfluorosulfonimide, la bistrifluorométhanesulfonimide, le bistrifluorométhanesulfonylméthane, le tristrifluorométhanesulfonylméthane et le trisfluorosulfonylméthane.

Les mélanges binaires comprenant les composants (a) et (b) ci-dessus sont des conducteurs protoniques anhydres. En effet, une base azotée protonée est capable de transférer son proton à une base azotée non-protonée suivant un mécanisme de Grotthus, permettant le déplacement du proton dans ces milieux. Par exemple, dans le cas du mélange binaire imidazole/triflate d'imidazolium à la composition eutectique (3:1 molaire), la conductivité de ce mélange est supérieure à $10^{-3}\ \Omega^{-1}.cm^{-1}$ à 25°C. Un tel mélange constitue ainsi un conducteur protonique anhydre neutre présentant une conductivité élevée.

Les conducteurs protoniques selon l'invention sont particulièrement intéressant pour les systèmes de modulation de la lumière, dits électrochromes. Les systèmes électrochromes utilisent généralement des films de semi-conducteurs à large bande interdite, tels que $H_xWO_3$ ou $IrO_2H_x$, $H_xTiO_2$, $H_xTa_2O_5$, $H_xMnO_2$, $H_xCoO_2$, $H_xNiO_2$, x étant typiquement compris entre 0 et 0,4, dans lesquels l'injection ou extraction concomitante de protons et d'électrons s'accompagne d'une variation de l'absorption ou de la réflectance optique, dans le visible ou l'infrarouge.

D'autres systèmes électrochromes utilisent des précurseurs solubles de radicaux libres fortement colorés comme les viologènes ("Weitz blue") dont la coloration s'obtient par réduction, ou les tétraalkyl-1,4 aryl-diamines ("Wurster blue") dont la coloration s'obtient par oxydation. Cependant, ces composés à l'état coloré ne présentent qu'une stabilité limitée, en particulier vis-à-vis de la lumière et de l'oxygène et ne peuvent être utilisés dans les vitrages ou systèmes d'affichage exposés à la lumière naturelle.

Les conducteurs protoniques selon l'invention permettent de mettre en oeuvre un principe nouveau de formation réversible d'espèces colorées, par réactions électrochimiques impliquant l'injection de deux électrons et d'un ou deux protons, comme suit:

$$[ox]^{+} + 2e^{-} + H^{+} \rightarrow [red]$$

$$[ox]^- + 2e^- + 2H^+ \rightarrow [red]^-$$

$$[ox] + 2e^- + 2H^+ \rightarrow [red]$$

les passages de *ox* à *red* correspondant à un changement de couleur. Dans ce cas, les espèces colorées ne sont pas des radicaux et présentent une stabilité nettement améliorée par rapport aux systèmes radicalaires. Les conducteurs protoniques selon l'invention ont non seulement un pouvoir de protonation élevé, un pH contrôlé par le choix de l'hétéroatome de type azole, mais aussi, par rapport aux milieux aqueux, une plage de température de fonctionnement nettement plus large et un pouvoir solubilisant des molécules organiques.

La présente invention a donc également pour objet un système électrochrome comprenant deux électrodes semi-conductrices transparentes disposées de façon opposée et espacée l'une par rapport à l'autre, chacune des électrodes étant fixée d'un côté à un support transparent et comportant de l'autre côté un revêtement d'un semi-conducteur à large bande interdite, et un électrolyte polymère tel que défini ci-dessus disposé entre les électrodes et contactant les revêtements de semi-conducteur.

L'invention vise aussi un système électrochrome comprenant deux électrodes semi-conductrices transparentes disposées de façon opposée et espacée l'une par rapport à l'autre, chacune des électrodes étant fixée d'un côté à un support transparent et un électrolyte polymère tel que défini ci-dessus disposé entre les électrodes et contactant l'autre côté de chaque électrode, l'électrolyte polymère étant additionné d'au moins un colorant rédox.

L'invention vise également un système électrochrome caractérisé par le fait qu'il comprend deux électrodes semi-conductrices transparentes disposées de façon opposée et espacée l'une par rapport à l'autre, chacune des électrodes étant fixée d'un côté à un support transparent et une des électrodes comportant de l'autre côté un revêtement d'un semi-conducteur à large bande interdite, et un électrolyte polymère tel que défini ci-dessus, disposé entre les électrodes et contactant le revêtement de semi-conducteur, l'électrolyte polymère étant additionné d'au moins un couple rédox complémentaire du semi-conducteur à large bande interdite.

À titre d'exemples non limitatifs, les molécules susceptibles de subir des transformations de couleur réversibles sont choisies parmi les suivantes :

| forme réduite | forme oxydée |
|---|---|
| 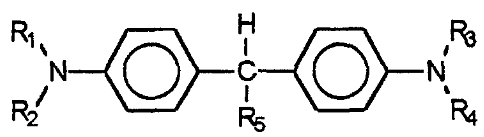 | 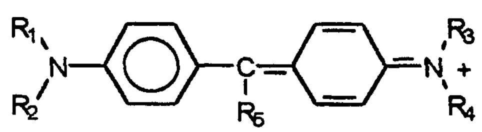 |
| 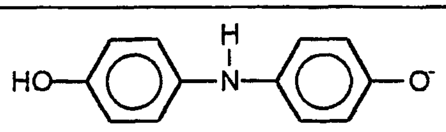 | 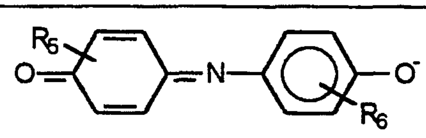 |

| | |
|---|---|
| 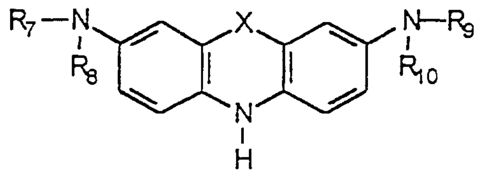 | 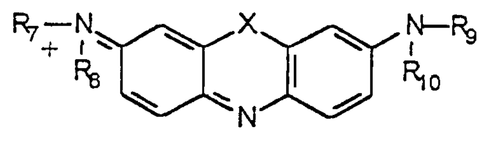 X = S, O, $NR_{11}$ |
| 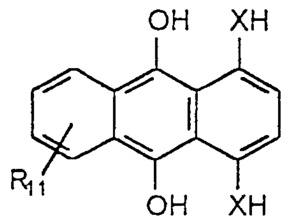 | 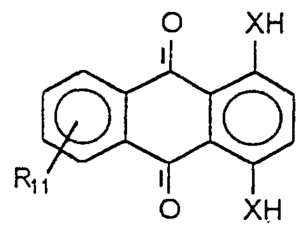 |
| 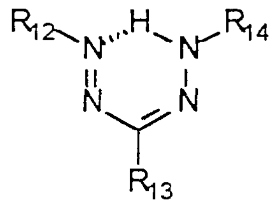 | 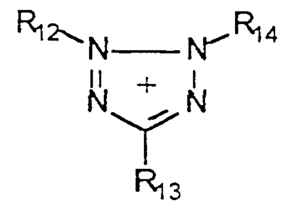 |
| 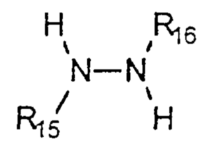 | 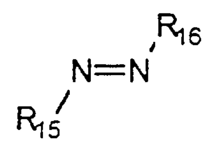 |

Dans les formules ci-dessus, $R_1$ à $R_{10}$ sont des groupements alkyle, aryle, arylalkyle ou alkylaryle comportant éventuellement des substituants oxa, aza, thia ou des halogènes, des groupements nitro, cyano, carboxylate, sulfonate, onium, dans les chaînes alkyles ou dans les cycles.

$R_{11}$ à $R_{16}$ sont des groupements aryle, alkylaryle, hétérocyclique, comportant éventuellement des substituants oxa, aza, thia ou des halogènes, des groupements nitro, cyano, carboxylate, sulfonate, onium, dans les chaînes alkyles ou dans les cycles.

À titre d'exemples de colorants ou couples rédox permettant la modulation de la coloration par application d'un courant, on peut mentionner:

- leuco(cristal violet) ↔ cristal violet (forme oxydée violette),
- leuco(vert malachite) ↔ vert malachite (forme oxydée verte),
- leuco(bleu de méthylène) ↔ bleu de méthylène (forme oxydée bleu),
- bis(4-hydroxyphénylamine) ↔ sel d'imidazolium de l'indophénol (forme oxydée rouge),
- 3,4-dihydroxy-9,10-anthraquinone (alizarine, forme réduite rouge)↔ 3,4,9,10-tétrahydroxyanthracène,
- 4,4'bis(4-nitrophénylformazan)-3,3'-méthoxybiphényl (forme réduite bleu) ↔ tétranitrotétrazolium blue cation,
- triphényl formazan (forme réduite bleu) ↔ 2,3,5 triphényl-2H-tétrazolium cation,
- 1,4-diaminophényl-hydrazino-1-naphtalène ↔ 1,4-diaminophényl-azo-1-naphtalène (Fat brown RR, forme oxydé marron).

Les couples rédox peuvent être associés d'une manière complémentaire, c'est-à-dire que l'un des réactifs se colore par réduction alors que son complément se colore par oxydation. Il est aussi possible d'inclure dans le milieu des molécules subissant des réactions rédox sans changer de couleur, permettant la réaction de formation du colorant à une seule électrode. On peut citer par exemple les composés mettant en jeu la formation et la scission de liaisons disulfure tel le dimercaptothiadiazole, le mercaptométhyltétrazole dont les anions correspondants sont stables dans le milieu électrolytique de l'invention.

Il est aussi possible d'associer aux colorants des familles précitées des molécules organométalliques présentant des couples rédox stables tel le férrocène ou ses dérivés, ou encore d'utiliser des contre-électrode sur lesquelles un semi-conducteur à large bande interdite, tel que $H_xWO_3$, $IrO_2H_x$, $H_xTiO_2$, $H_xTa_2O_5$, $H_xMnO_2$, $H_xNiO_2$, $H_xCoO_2$ (x étant typiquement compris entre 0 et 0,4), est déposé.

Les conducteurs protoniques selon l'invention sont aussi intéressants pour les générateurs électrochimiques, les supercapacités électrochimiques comprenant au moins une électrode de carbone, ou au moins une électrode d'oxyde métallique ou au moins un polymère à propriétés rédox. Les conducteurs protoniques selon l'invention peuvent être utilisés comme solvants protiques anhydres présentant un fort pouvoir solvatant et également comme solvants pour effectuer des réactions chimiques, photochimiques ou électrochimiques.

En plus de tous les avantages ci-haut mentionnés, les conducteurs protoniques selon la présente invention forment des électrolytes polymères lorsqu'ils sont mélangés avec des polymères contenant des groupes polaires, comme les polyéthers, les polyesters, le polyéthylèneimine, le polyacrylonitrile, le polyfluorure de vinylidène, ou le polyvinyl butyrale. On obtient ainsi des menbranes possédant à la fois une bonne tenue mécanique et une conductivité élevée. En outre, la conductivité des conducteurs protoniques liquides, gels ou polymères selon la présente invention peut être augmentée en ajoutant au moins un solvant polaire peu volatil comme la diméthylformamide, la diméthylacétamide, les . tétraalkylsulfamides ou les glymes.

## Description Sommaire des Dessins

D'autres avantages et caractéristiques de l'invention apparaîtront plus clairement à la lecture de la description ci-après de modes de réalisation préférés, faite en référence aux dessins ci-joints, dans lesquels:

- la Figure 1 représente une section schématique partielle d'un système électrochrome comprenant un électrolyte polymère selon l'invention;
- la Figure 2 représente une section schématique partielle d'un autre système électrochrome comprenant un électrolyte polymère selon l'invention;
- la Figure 3 représente le diagramme de phase du mélange binaire imidazole/triflate d'imidazolium;
- la Figure 4 représente le diagramme de phase du mélange binaire imidazole/bis(trifluorométhanesulfonimide) d'imidazolium;
- la Figure 5 représente les diagrammes de phase du mélange binaire imidazole/triflate d'imidazolium, en l'absence et en présence de poly(oxyde d'éthylène) ou poly(éthylène glycol);
- la Figure 6 est un diagramme représentant la variation de la conductivité du mélange binaire imidazole/triflate d'imidazolium en fonction de la température, comparée à d'autres électrolytes;
- la Figure 7 représente la courbe de décharge obtenue à partir d'un générateur électrochimique comprenant un mélange binaire triazole/bisfluorosulfonimide de triazolium; et
- la Figure 8 représente la courbe de cyclage obtenue à partir d'un système électrochrome comprenant un électrolyte polymère formé du mélange binaire 1,2,3-triazole/bis(trifluorométhanesulfonimide) de 1,2,3-triazolium dissout dans le poly(oxyde d'éthylène).

## Manières de Réaliser l'Invention

Le système électrochrome illustré à la Figure 1 comprend deux électrodes 10 semi-conductrices transparentes disposées de façon opposée et espacée l'une par rapport à l'autre, chacune des électrodes 10 étant fixée d'un côté à un support 12 transparent tel que du verre et comportant de l'autre côté un revêtement 14 d'un semi-conducteur à large bande interdite. Un électrolyte polymère 16 conforme à l'invention est disposé entre les électrodes 10 et contacte les revêtements 14 de semi-conducteur. Le système électrochrome illustré à la Figure 2 est similaire à celui de la Figure 1, à l'exception que les électrodes 10 ne sont pas recouverts d'un revêtement 14, l'électrolyte polymère 16' étant plutôt additionné de deux colorants rédox complémentaires. L'application d'un potentiel aux électrodes 10 engendre un changement de coloration des revêtements 14 dans le cas du système électrochrome de la Figure 1 et un changement de coloration des colorants rédox présents dans l'électrolyte polymère protonique 16' dans le cas du système électrochrome de la Figure 2.

La Figure 3 représente le diagramme de phase du mélange binaire imidazole/triflate d'imidazolium. Tel qu'illustré, ce mélange binaire présente un palier eutectique pour un rapport molaire imidazole/sel (I/S) égal à 3, d'un point de fusion d'environ 12°C. La Figure 4, d'autre part, représente le diagramme de phase du mélange binaire imidazole/bistrifluorométhanesulfonimide d'imidazolium. Tel qu'illustré à la Figure 4, ce mélange binaire présente un palier eutectique pour un rapport molaire I/S égal à 4, d'un point de fusion d'environ -10°C. De la même manière, le mélange binaire imidazole/bisfluorosulfonimide d'imidazolium à sa composition eutectique (rapport molaire I/S égal à 4) a un point de fusion inférieur à -20°C. La nature des substituants influe également sur les propriétés de conducteurs liquides; ainsi, pour le mélange binaire 2-hexylimidazole/triflate de 2-hexylimidazolium, la température du palier eutectique pour un rapport molaire I/S égal à 3 ou 4 est inférieur à -10°C.

La Figure 5 illustre le fait que l'adjonction de 20% en poids de poly(oxyde d'éthylène) à un mélange binaire imidazole/triflate d'imidazolium ayant un rapport molaire I/S égal à 4 modifie peu le diagramme de phase du système alors que le système se présente maintenant sous la forme d'un film d'électrolyte polymère particulièrement commode pour la réalisation de systèmes électrochimiques en film mince : systèmes de modulation de la lumière tels que décrit précédemment, générateurs, supercapacités, capteurs, etc. La Figure 6 donne la conductivité de cette électrolyte polymère protonique et permet de comparer sa conductivité à celle d'autres électrolytes polymères conducteurs protoniques : un électrolyte obtenu par mélange d'acide orthophosphorique et de poly(oxyde d'éthylène) (POE/$H_3PO_4$), un électrolyte obtenu par mélange d'acide orthophosphorique et de polyéthylèneimine (PEI/$H_3PO_4$), un électrolyte obtenu par mélange de sulfamide et de poly(oxyde d'éthylène) (POE/sulfamide). Enfin, la conductivité des électrolytes liquides ou polymères obtenus à partir des mélanges binaires conformes à l'invention peut être augmenté en ajoutant un solvant polaires peu volatils comme de la diméthylformamide ou des glymes.

Les exemples non-limitatifs suivants illustrent l'invention.

**Exemple 1**

A une solution dans 15 ml d'éther de 1,36 g (20 mmoles) d'imidazole, on a ajouté 5,62 g (20 mmoles), de bistrifluorométhanesulfonimide $(CF_3SO_2)_2NH$ (commercialisé par Fluka), puis après 1 heure sous agitation, on a récupéré le précipité obtenu par filtration sur un verre fritté de porosité N°3. Après séchage, on a récupéré quantitativement le sel de bistrifluorométhanesulfonimide d'imidazolium de formule:

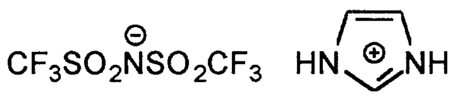

Le broyage en boîte à gants d'un mélange molaire de quatre moles d'imidazole pour une mole de sel d'imidazolium a permis d'obtenir un conducteur liquide ayant une température de fusion inférieure à l'ambiante. Ce conducteur liquide présente une conductivité protonique élevée proche de $10^{-3}$ $S.cm^{-1}$ à 20°C.

**Exemple 2**

En boîte à gants sous argon, on a préparé une solution dans 50 ml de formiate de méthyle de 6,81 g (100 mmoles) d'imidazole, de 5,62 g (20 mmoles) de bistrifluorométhanesulfonimide $(CF_3SO_2)_2NH$ (commercialisé par Fluka) et de 2,49 g de poly(oxyde d'éthylène) de masse $M_W = 5.10^6$ (20% en poids relativement au sel). Cette solution a ensuite été épandue sur un film de polypropylène. Après 24 heures en boîte à gants, on a obtenu un film d'électrolyte polymère transparent et présentant de bonnes propriétés mécaniques.

**Exemple 3**

On a réalisé, en boîte à gants sous argon, un générateur électrochimique en film mince, de technologie polymère, utilisant le mélange binaire imidazole/bistrifluorométhanesulfonimide d'imidazolium à sa composition eutectique (4:1 molaire) comme électrolyte, en superposant les couches suivantes :

- une anode constituée par un mélange de dioxyde de titane $TiO_2$ anatase (45% en volume), de noir de Shawinigan (5% en volume), de l'électrolyte liquide ci-dessus (40% en volume) et de poly(oxyde d'éthylène) de masse $M_w = 3.10^5$ (10% en volume) déposé sur un collecteur de courant en acier inoxydable;
- une cathode constituée par un mélange similaire à l'anode mais en substituant $TiO_2$ par du dioxyde de manganèse $MnO_2$, préalablement réduit par un équivalent d'hydrazine, et déposé sur un collecteur de courant en acier inoxydable; et
- un film d'électrolyte constitué par un mélange de l'électrolyte liquide ci-dessus (80% en poids) et de poly(oxyde d'éthylène) (20% en poids).

Après assemblage, l'ensemble a été scellé dans un boitier de pile de bouton. Ce générateur électrochimique initialement à l'état déchargé a été cyclé entre 1,2 V et 500 mV à un régime de charge/décharge de C/10. On a ainsi réalisé une centaine de cycles en conservant 90% de la capacité à la première décharge. Ce générateur a une densité d'énergie pratique de l'ordre de 100 Wh/kg, la densité d'énergie calculée en ne tenant compte que des matériaux électroactifs étant de 160 Ah/kg ou 709 Ah/1.

On a également réalisé un générateur électrochimique utilisant le mélange binaire imidazoline/bistrifluorométhanesulfonimide d'imidazolinium à sa composition eutectique qui a donné des performances similaires.

**Exemple 4**

Dans une extrudeuse Warner & Pfilder sous atmosphère d'argon et opérant dans une chambre anhydre, on a introduit à une extrémité du poly(oxyde d'éthylène) de masse $M_w = 3.10^5$ sous forme de pastilles de 2 mm de diamètre et dans l'extrudeuse un mélange d'imidazole, de bistrifluorométhanesulfonimide de 2-hexylimidazolium, du fluorure de fer hydrogéné $H_xFeF_3$ broyé en grains de taille inférieure à 5 μm, du noir de Shawinigan, du KETJENBLACK® K600 (marque de commerce, vendu par la Société AKZO) et des particules de silice (vendu sous la marque de commerce AEROSIL R974 par la Société Degussa). L'ensemble des composants ont été introduits dans des proportions telles que $H_xFeF_3$ représente 45% du volume totale, le noir de Shawinigan 3%, le KETJENBLACK® K600 1%, les particules de silice 1%, le poly(oxyde d'éthylène) 5% et les autres composants représentant 45% du volume totale ; le rapport molaire imidazole/bistrifluorométhanesulfonimide de 2-hexylimidazolium étant de 4 à 1. L'ensemble a alors été extrudé à une température de 100°C sous la forme d'une bande de 14 cm de large et d'une épaisseur de 120 μm, ce film de cathode étant directement déposé sur une feuille d'acier inoxydable de 8 μm d'épaisseur.

Au cours du procédé, ce film de cathode composite a lui-même été recouvert d'un film d'électrolyte de 30 μm d'épaisseur obtenu par extrusion d'un mélange de poly(oxyde d'éthylène) de masse $M_w = 9.10^5$ (17% en poids), de particules de silice (AEROSIL R974) et d'un mélange d'imidazole et de bistrifluorométhanesulfonimide de 2-hexylimidazolium (80% poids, rapport molaire 4:1).

Au cours du procédé, ce film d'électrolyte déposé sur la cathode a lui-même été recouvert d'un film d'anode obtenu dans les mêmes conditions que le film de cathode mais en substituant $H_xFeF_3$ par du $TiO_2$ anatase. L'ensemble alors été laminé avec une feuille d'acier inoxydable de 8 μm d'épaisseur.

Ce générateur électrochimique initialement à l'état déchargé a été cyclé entre 1,2 V et 500 mV à un régime de charge/décharge de C/10. On a ainsi réalisé 1000 cycles en conservant 78% de la capacité à la première décharge.

**Exemple 5**

On a réalisé un générateur électrochimique similaire à celui décrit dans l'exemple 3, mais utilisant d'une part des matériaux électroactifs organiques, soit une anode à base d'anthrahydroquinone (oxanthranol) et une cathode à base de tétrachloroquinone (chloranil), et d'autre part un mélange binaire triazole/bisfluorosulfononimide, à sa composition eutectique, gélifié par du polyfluorure de vinylidène (commercialisé par la société Montedison) en lieu et place du poly(oxyde d'éthylène).

La courbe de décharge obtenue à un régime de décharge de C/30 est donnée sur la Figure 7. Sur cette figure, le potentiel du générateur, U, exprimé en mV est donnée en ordonnée, et le taux d'utilisation des matériaux électroactifs, x, exprimé en % est donnée en abscisse.

Ce générateur a une densité d'énergie calculée en ne tenant compte que des matériaux électroactifs de 120 Ah/kg.

**Exemple 6**

On a réalisé une supercapacité électrochimique utilisant des électrodes à base de dioxyde de ruthénium $RuO_2$. Ce type de supercapacité met à profit le phénomène de pseudo-insertion du proton dans ces oxydes. Les électrodes ont été obtenues par déposition sous vide. On a assemblé une supercapacité utilisant deux de ces électrodes et un électrolyte constitué du mélange binaire 1,3,4-triazole/trisfluorosulfonylméthylure de 1,3,4-triazolium à sa composition eutectique.

Cette supercapacité a été cyclée entre 0 et 1 V à un régime de charge/décharge de 10C. On a pu effectuer dans ces conditions plus de 300000 cycles, la capacité au 300000$^{éme}$ cycles étant encore égale à 70% de la capacité du premier cycle. Cette supercapacité présente une densité d'énergie supérieure à 5 Wh/kg et peut fournir une puissance supérieure à 1 kW/kg.

**Exemple 7**

On a réalisé une supercapacité électrochimique utilisant des électrodes à base de carbone activé. Ces électrodes étaient des composites obtenus à partir de fibre de carbone et de fibre d'aluminium en milieu réducteur. Les électrodes d'une épaisseur de 150 μm ont été placés de part et d'autre d'un polyéthylène microporeux d'une épaisseur de 40 μm, l'ensemble a été imprégné avec le mélange binaire pyrazole/bis(trifluorométhanesulfonyl)méthylure de pyrazolium à sa composition eutectique, puis scellé en boîte à gants dans un boitier de pile bouton. On a obtenu de

bonnes performances avec cette supercapacité, on a ainsi pu réaliser plus de 200000 cycles de charge/décharge entre 0 et 1 V pour une densité d'énergie supérieure à 10 Wh/l et une puissance délivrée supérieure à 1500 W/l.

**Exemple 8**

On a réalisé un système électrochrome, en boîte à gants sous argon, utilisant le mélange binaire 1,2,3-triazole/bistrifluorométhanesulfonimide de 1,2,3-triazolium à sa composition eutectique (rapport molaire 4:1) comme électrolyte, en superposant les couches suivantes :

- une électrode transparente constituée par le dépôt sur une plaque de verre d'une couche d'oxyde d'iridium hydrogéné $H_xIrO_2$ et d'une sous couche conductrice d'oxyde d'étain;
- un film d'électrolyte polymère transparent constitué par un mélange de l'électrolyte liquide ci-dessus (80% en poids) et de poly(oxyde d'éthylène) (20% en poids); et
- une électrode transparente constituée d'une couche de trioxyde de tungstène $WO_3$ et d'une sous-couche conductrice d'oxyde d'étain.

Cette électrochrome a permis d'obtenir une variation de l'absorption optique variant de 80% à l'état décoloré à 30% à l'état coloré.

La courbe de cyclage obtenue avec l'électrochrome ci-dessus à un régime de charge/décharge de 10C est donnée sur la Figure 8. Sur cette figure, la capacité coulombique relativement à celle du premier cycle, C, exprimé en % est donnée en ordonnée, et le nombre de cycles, n, exprimé en milliers de cycle est donnée en abscisse.

**Exemple 9**

On a réalisé un électrochrome en dissolvant deux colorants complémentaires dans le mélange binaire imidazole/bistrifluorométhanesulfonimide d'imidazolium à sa composition eutectique. En boîte à gants, on a cobroyé 1,75 g (5 mmoles) de bistrifluorométhanesulfonimide d'imidazolium avec 1,36 g (20 mmoles) d'imidazole. Puis on a ajouté au mélange binaire 16,5 mg (50 μmoles) de leuco (vert malachite), à l'état réduit incolore, et 30,8 mg (50 μmoles) de bistrifluorométhanesulfonimide de 3-(4,5-diméthylthiazolyl-2-yl)-2,5-diphényl-2H-tétrazolium (MTT), à l'état oxydé incolore (obtenu par échange ionique dans l'eau à partir du bromure). On a alors ajouté 8% en poids de particules de silice (AEROSIL R 974). Le liquide visqueux obtenu a été déposé entre deux plaques de verre recouverte d'une couche conductrice d'oxyde d'étain et d'indium (ITO). Après avoir scellé l'ensemble pour le rendre étanche, on a appliqué à l'extérieur un potentiel de 1300 mV à l'aide d'un potentiostat. Le système s'est alors coloré, la forme oxydé de la leuco (malachite verte) et la forme réduite du MTT présentant chacune une bande d'absportion intense dans le visible. En appliquant un potentiel de -500 mV, on a pu observé une décoloration relativement rapide du système (inférieur à 60 s). Un tel système électrochrome, présentant une durée de vie importante, est facile à mettre en oeuvre, même pour des systèmes de grande taille (supérieure au $m^2$) qui utilisent aussi bien du verre qu'un polymère convenablement traité comme électrode transparente conductrice. De plus, l'énergie nécessaire pour conserver la coloration est relativement faible, inférieure à 1 $W/m^2$.

**Exemple 10**

On a réalisé un système électrochrome similaire à celui décrit dans l'exemple 9 mais utilisant comme colorants la N,N,N',N'-tétraméthylphénylène-diamine (état réduit incolore) et un bistrifluorométhanesulfonimide de diméthylviologène (état oxydé incolore). Ce système électrochrome se colore aussi en appliquant un potentiel aux deux électrodes. De façon surprenante, ce système présente une stabilité importante malgré le mécanisme radicalaire mis en jeu lors de la coloration. L'électrolyte liquide en plus de solubiliser les colorants permet de stabiliser les radicaux.

**Exemple 11**

On a réalisé une supercapacité électrochimique en utilisant le mélange binaire triazole/bistrifluorométhanesulfonimide de triazole et des électrodes d'oxyde de Ruthénium RuO2.

**Exemple 12**

On a réalisé une supercapacité électrochimique en utilisant le mélange binaire imidazolium/tris(fluorosulfonyl) méthylure d'imidazolium et des électrodes d'oxyde de carbone activé et en ajoutant un glyme comme plastifiant.

**Exemple 13**

On a réalisé un système électrochrome HxIrO2/WO3 en utilisant le mélange binaire triazole/triflate de triazolium additionné d'un diacrylate de poly(oxyde d'éthylène) et d'un sensibilisateur (Irgacure), et en réticulant le tout en U.V.

**Exemple 14**

On a réalisé un électrochrome à colorants en utilisant deux colorants complémentaires dissous dans un sel de tristrifluorométhanesulfonylméthylure.

## Revendications

1. Conducteur protonique, caractérisé par le fait qu'il est constitué d'un mélange des composants (a) et (b) suivants:

   a) un sel d'addition d'une base azotée avec un acide, répondant à la formule:

   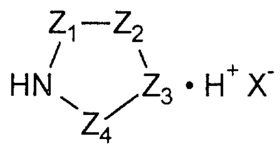

   dans laquelle:

   $Z_1$, $Z_2$, $Z_3$ et $Z_4$, identiques ou différents, représentent un groupement -N= ou -C($Y_i$)= dans lequel $Y_i$ désigne un atome d'hydrogène, un radical alkyle linéaire ou ramifié ayant de 1 à 20 atomes de carbone, un radical fluoroalkyle ayant de 1 à 20 atomes de carbone, ou un radical oxoalkyle ou azaalkyle ayant de 1 à 20 atomes de carbone, sous réserve qu'au moins un et au plus deux des groupements $Z_1$, $Z_2$, $Z_3$ et $Z_4$ représente -N=, deux atomes de carbone adjacents étant éventuellement hydrogénés et la base azotée faisant éventuellement partie d'une trame polymérique, et
   $X^-$ représente un anion dérivé d'un acide choisi dans le groupe constitué par les acides sulfoniques de formule $R_FSO_3H$, les sulfonimides de formule $(R_FSO_2)(R'_FSO_2)NH$ et les méthylures de formule $(R_FSO_2)(R'_FSO_2)CH_2$ ou $(R_FSO_2)(R'_FSO_2)(R''_FSO_2)CH$, dans lesquelles $R_F$, $R'_F$ ou $R''_F$ représentent indépendamment un radical $F(CF_2)_n$-, n étant compris entre O et 6, l'acide faisant éventuellement partie d'une trame polymérique; et

   b) une base azotée répondant à la formule:

   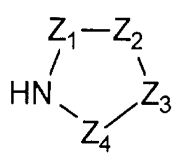

   dans laquelle $Z_1$, $Z_2$, $Z_3$ et $Z_4$ ont les significations précitées, la base azotée faisant éventuellement partie d'une trame polymérique;

   les composants (a) et (b) étant présents dans des proportions telles à former une composition ayant un point de fusion inférieure à 25°C.

2. Conducteur protonique selon la revendication 1, caractérisé par le fait que le composant (a) est un sel d'addition d'une base azotée avec un acide, dont la base azotée est un imidazole répondant à la formule:

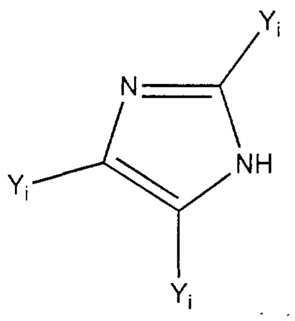

dans laquelle $Y_i$ a la signification précitée.

**3.** Conducteur protonique selon la revendication 1, caractérisé par le fait que le composant (a) est un sel d'addition d'une base azotée avec un acide, dont la base azotée est un 1,2,3-triazole répondant à la formule:

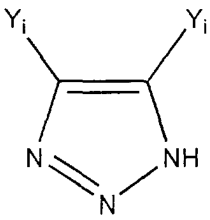

dans laquelle $Y_i$ a la signification précitée.

**4.** Conducteur protonique selon la revendication 1, caractérisé par le fait que le composant (a) est un sel d'addition d'une base azotée avec un acide, dont la base azotée est un 1,3,4-triazole répondant à la formule:

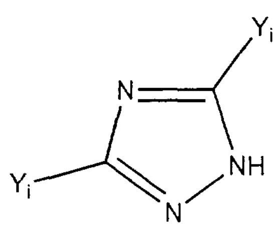

dans laquelle $Y_i$ a la signification précitée.

**5.** Conducteur protonique selon la revendication 1, caractérisé par le fait que le composant (a) est un sel d'addition d'une base azotée avec un acide, dont la base azotée est un pyrazole répondant à la formule:

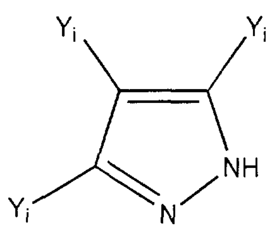

dans laquelle $Y_i$ a la signification précitée.

**6.** Conducteur protonique selon la revendication 1, caractérisé par le fait que le composant (a) est un sel d'addition d'une base azotée avec un acide, dont la base azotée est une imidazoline répondant à la formule:

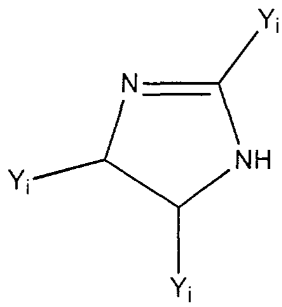

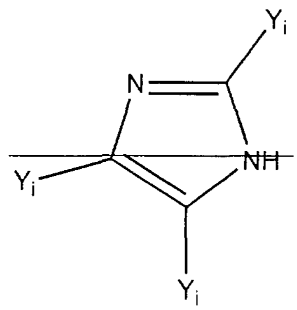

dans laquelle $Y_i$ a la signification précitée.

**7.** Conducteur protonique selon l'une des revendications 1 à 6, caractérisé par le fait que l'acide est choisi dans le groupe constitué par l'acide triflique, la bisfluorosulfonimide, la bistrifluorométhanesulfonimide, le bistrifluorométhanesulfonylméthane, le tristrifluorométhanesulfonylméthane et le trisfluorosulfonylméthane.

**8.** Conducteur protonique selon la revendication 2, caractérisé par le fait que le composant (a) est un sel d'addition de l'imidazole avec l'acide triflique.

**9.** Conducteur protonique selon la revendication 2, caractérisé par le fait que le composant (a) est un sel d'addition de l'imidazole avec la bisfluorosulfonimide.

**10.** Conducteur protonique selon la revendication 2, caractérisé par le fait que le composant (a) est un sel d'addition de l'imidazole avec la bistrifluorométhanesulfonimide.

**11.** Conducteur protonique selon la revendication 2, caractérisé par le fait que le composant (a) est un sel d'addition du 2-hexylimidazole avec l'acide triflique.

**12.** Conducteur protonique selon l'une des revendications 1 à 11, caractérisé par le fait que le composant (b) est une base azotée constituée par un imidazole répondant à la formule:

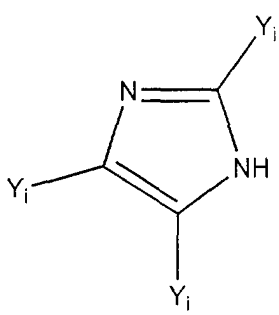

dans laquelle $Y_i$ a la signification précitée.

**13.** Conducteur protonique selon l'une des revendications 1 à 11, caractérisé par le fait que le composant (b) est une base azotée constituée par un 1,2,3-triazole répondant à la formule:

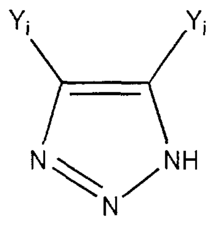

dans laquelle $Y_i$ a la signification précitée.

**14.** Conducteur protonique selon l'une des revendications 1 à 11, caractérisé par le fait que le composant (b) est une base azotée constituée par un 1,3,4-triazole répondant à la formule:

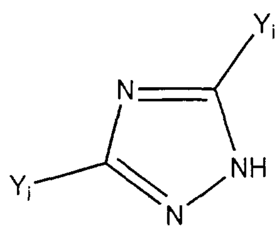

dans laquelle $Y_i$ a la signification précitée.

**15.** Conducteur protonique selon l'une des revendications 1 à 10, caractérisé par le fait que le composant (b) est une base azotée constituée par un pyrazole répondant à la formule:

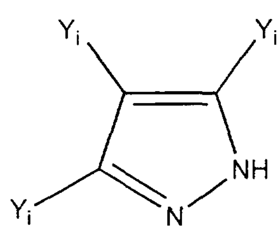

dans laquelle $Y_i$ a la signification précitée.

**16.** Conducteur protonique selon l'une des revendications 1 à 11, caractérisé par le fait que le composant (b) est une base azotée constituée par une imidazoline répondant à la formule:

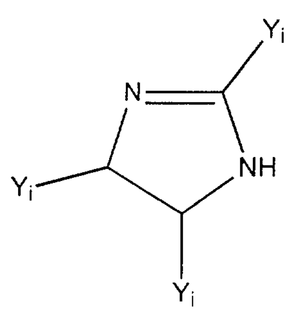

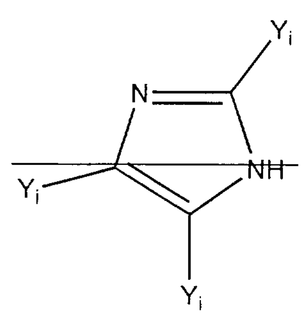

dans laquelle $Y_i$ a la signification précitée.

**17.** Conducteur protonique selon l'une des revendications 1 à 11, caractérisé par le fait que le composant (b) est une base azotée constituée par l'imidazole.

**18.** Conducteur protonique selon l'une des revendications 1 à 11, caractérisé par le fait que le composant (b) est une base azotée constituée par le 2-hexylimidazole.

**19.** Conducteur protonique selon la revendication 1, caractérisé par le fait qu'il est constitué d'un mélange de 3 moles d'imidazole et d'une mole de triflate d'imidazolium.

**20.** Conducteur protonique selon la revendication 1, caractérisé par le fait qu'il est constitué d'un mélange de 4 moles d'imidazole et d'une mole de bis(trifluorométhanesulfonimide) d'imidazolium.

**21.** Conducteur protonique selon la revendication 1, caractérisé par le fait qu'il est constitué d'un mélange de 4 moles d'imidazole et d'une mole de bisfluorosulfonimide d'imidazolium.

**22.** Conducteur protonique selon la revendication 1, caractérisé par le fait qu'il est constitué d'un mélange de 3 ou 4 moles de 2-hexylimidazole et d'une mole de 2-hexylimidazolium.

**23.** Electrolyte liquide caractérisé par le fait qu'il est constitué par un conducteur protonique tel que défini dans l'une des revendications 1 à 22.

**24.** Electrolyte polymère caractérisé par le fait qu'il comprend un conducteur protonique tel que défini dans l'une des revendications 1 à 22, dissout dans un polymère comportant au moins un groupement polaire.

**25.** Electrolyte polymère selon la revendication 24, caractérisé par le fait que le polymère est le poly(oxyde d'éthylène).

**26.** Système électrochrome caractérisé par le fait qu'il comprend deux électrodes semi-conductrices transparentes disposées de façon opposée et espacée l'une par rapport à l'autre, chacune desdites électrodes étant fixée d'un côté à un support transparent et comportant de l'autre côté un revêtement d'un semi-conducteur à large bande interdite, et un électrolyte polymère tel que défini dans la revendication 24 ou 25, disposé entre lesdites électrodes et contactant les revêtements de semi-conducteur.

**27.** Système électrochrome caractérisé par le fait qu'il comprend deux électrodes semi-conductrices transparentes disposées de façon opposée et espacée l'une par rapport à l'autre, chacune desdites électrodes étant fixée d'un côté à un support transparent et un électrolyte polymère tel que défini dans la revendication 24 ou 25, disposé entre lesdites électrodes et contactant l'autre côté de chaque électrode, ledit électrolyte polymère étant additionné d'au moins un colorant rédox.

**28.** Système électrochrome caractérisé par le fait qu'il comprend deux électrodes semi-conductrices transparentes disposées de façon opposée et espacée l'une par rapport à l'autre, chacune desdites électrodes étant fixée d'un côté à un support transparent et une desdites électrodes comportant de l'autre côté un revêtement d'un semi-conducteur à large bande interdite, et un électrolyte polymère tel que défini dans la revendication 24 ou 25, disposé entre lesdites électrodes et contactant le revêtement de semi-conducteur, ledit électrolyte polymère étant additionné d'au moins un couple rédox complémentaire dudit semi-conducteur à large bande interdite.

**29.** Générateur électrochimique composé d'une anode, d'une cathode et d'un électrolyte tel que défini dans l'une des revendications 23 à 25, disposé entre l'anode et la cathode.

**30.** Supercapacité électrochimique composée d'une anode, d'une cathode et d'un électrolyte tel que défini dans l'une des revendications 23 à 25, disposé entre l'anode et la cathode.

**31.** Capteur comprenant un conducteur protonique tel que défini dans l'une des revendications 1 à 22.

**32.** Solvant protique anhydre caractérisé par le fait qu'il est constitué d'un conducteur protonique tel que défini dans l'une des revendications 1 à 22.

**33.** Utilisation d'un solvant protique anhydre tel que défini dans la revendication 32, pour effectuer des réactions chimiques, photochimiques ou électrochimiques.

**Patentansprüche**

**1.** Protonenleiter, umfassend ein Gemisch der nachstehend angeführten Komponenten (a) und (b):

a) ein Additionssalz einer Stickstoffbase mit einer Säure, emtsprechend der Formel

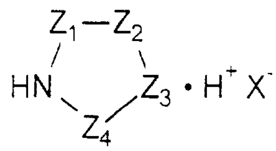

worin:

$Z_1$, $Z_2$, $Z_3$ und $Z_4$, die gleich oder unterschiedlich sein können, eine Gruppe -N = oder -C($Y_i$) = darstellen, worin $Y_i$ ein Wasserstoffatom, ein unverzweigter oder verzweigter Alkylrest mit 1 bis 20 Kohlenstoffatomen, ein Fluoralkylrest mit 1 bis 20 Kohlenstoffatomen oder ein Oxoalkylrest oder Azaalkylrest mit 1 bis 20 Kohlenstoffatomen ist, mit der Maßgabe, dass zumindest eine und höchstens zwei der Gruppen $Z_1$, $Z_2$, $Z_3$ und $Z_4$ -N= darstellen, wobei zwei benachbarte Kohlenstoffatome gegebenenfalls hydriert sind und die Stickstoffbase Teil einer Polymer-Grundeinheit sein kann, und

$X^-$ ein Anion darstellt, das von einer aus der aus Sulfonsäuren der Formel $R_FSO_3H$, Sulfonimiden der Formel $(R_FSO_2)(R'_FSO_2)NH$ und Methylestern der Formel $(R_FSO_2)(R'_FSO_2)CH_2$ oder $(R_FSO_2)(R'_FSO_2)(R''_FSO_2)CH$ bestehenden Gruppe ausgewählten Säure abgeleitet ist, worin $R_F$, $R'_F$ oder $R''_F$ unabhängig voneinander einen Rest $F(CF_2)_n$- darstellen, wobei n zwischen 0 und 6 liegt, und die Säure Teil einer Polymer-Grundeinheit sein kann, und

b) eine Stickstoffbase der Formel:

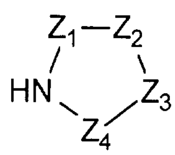

worin $Z_1$, $Z_2$, $Z_3$ und $Z_4$ wie oben definiert sind und die Stickstoffbase Teil einer Polymer-Grundeinheit sein kann;

wobei die Komponenten (a) und (b) in solchen Anteilen enthalten sind, dass sie eine Zusammensetzung mit einem Schmelzpunkt von unter 25 °C bilden.

**2.** Protonenleiter nach Anspruch 1, dadurch gekennzeichnet, dass Komponente (a) ein Additionssalz einer Stickstoffbase mit einer Säure ist, dessen Stickstoffbase ein Imidazol der Formel

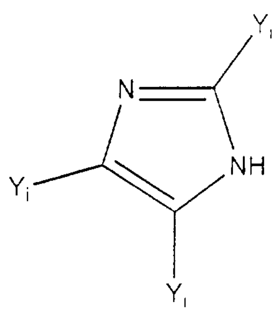

ist, worin $Y_1$ wie oben definiert ist.

**3.** Protonenleiter nach Anspruch 1, dadurch gekennzeichnet, dass Komponente (a) ein Additionssalz einer Stickstoffbase mit einer Säure ist, dessen Stickstoffbase ein 1,2,3-Triazol der Formel

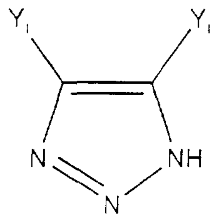

ist, worin $Y_1$ wie oben definiert ist.

**4.** Protonenleiter nach Anspruch 1, dadurch gekennzeichnet, dass Komponente (a) ein Additionssalz einer Stickstoffbase mit einer Säure ist, dessen Stickstoffbase ein 1,3,4-Triazol der Formel

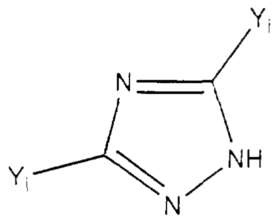

ist, worin $Y_i$ wie oben definiert ist.

**5.** Protonenleiter nach Anspruch 1, dadurch gekennzeichnet, dass Komponente (a) das Additionssalz einer Stickstoffbase mit einer Säure ist, dessen Stickstoffbase ein Pyrazol der Formel

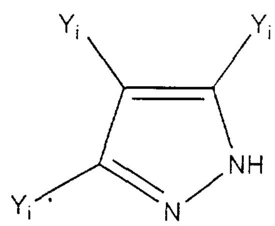

ist, worin $Y_i$ wie oben definiert ist.

**6.** Protonenleiter nach Anspruch 1, dadurch gekennzeichnet, dass Komponente (a) ein Additionssalz einer Stickstoffbase mit einer Säure ist, dessen Stickstoffbase ein Imidazolin der Formel

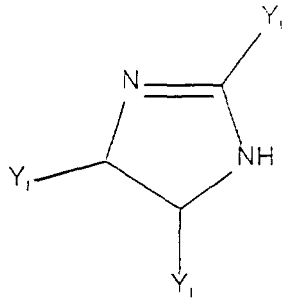

ist, worin $Y_i$ wie oben definiert ist.

7. Protonenleiter nach einem der Ansprüche 1 bis 6, dadurch gekennzeichnet, dass die Säure aus der aus Trifluoressigsäure, Bisfluorsulfonimid, Bistrifluormethansulfonimid, Bistrifluormethansulfonylmethan, Tristrifluormethansulfonylmethan und Trisfluorsulfonylmethan bestehenden Gruppe ausgewählt ist.

8. Protonenleiter nach Anspruch 2, dadurch gekennzeichnet, dass Komponente (a) ein Additionssalz von Imidazol mit Trifluoressigsäure ist.

9. Protonenleiter nach Anspruch 2, dadurch gekennzeichnet, dass Komponente (a) ein Additionssalz von Imidazol mit Bisfluorsulfonimid ist.

10. Protonenleiter nach Anspruch 2, dadurch gekennzeichnet, dass Komponente (a) ein Additionssalz von Imidazol mit Bistrifluormethansulfonimid ist.

11. Protonenleiter nach Anspruch 2, dadurch gekennzeichnet, dass Komponente (a) ein Additionssalz von 2-Hexylimidazol mit Trifluoressigsäure ist.

12. Protonenleiter nach einem der Ansprüche 1 bis 11, dadurch gekennzeichnet, dass Komponente (b) eine aus einem Imidazol der Formel

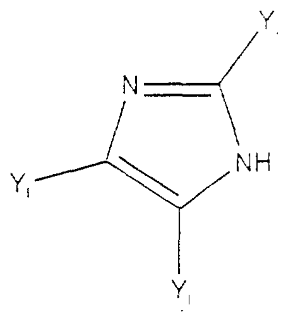

bestehende Stickstoffbase ist, worin $Y_i$ wie oben definiert ist.

13. Protonenleiter nach einem der Ansprüche 1 bis 11, dadurch gekennzeichnet, dass Komponente (b) eine aus einem 1,2,3-Triazol der Formel

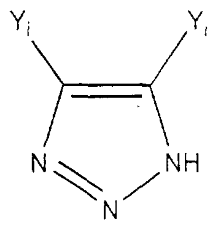

bestehende Stickstoffbase ist, worin $Y_1$ wie oben definiert ist.

**14.** Protonenleiter nach einem der Ansprüche 1 bis 11, dadurch gekennzeichnet, dass Komponente (b) eine aus einem 1,3,4-Triazol der Formel

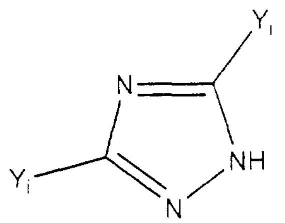

bestehende Stickstoffbase ist, worin $Y_i$ wie oben definiert ist.

**15.** Protonenleiter nach einem der Ansprüche 1 bis 10, dadurch gekennzeichnet, dass Komponente (b) eine aus einem Pyrazol der Formel

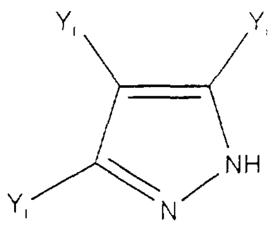

bestehende Stickstoffbase ist, worin $Y_i$ wie oben definiert ist.

**16.** Protonenleiter nach einem der Ansprüche 1 bis 11, dadurch gekennzeichnet, dass Komponente (b) eine aus einem Imidazolin der Formel

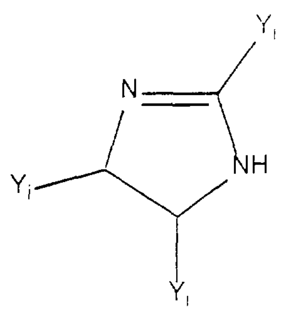

bestehende Stickstoffbase ist, worin $Y_i$ wie oben definiert ist.

**17.** Protonenleiter nach einem der Ansprüche 1 bis 11, dadurch gekennzeichnet, dass Komponente (b) eine aus Imidazol bestehende Stickstoffbase ist.

**18.** Protonenleiter nach einem der Ansprüche 1 bis 11, dadurch gekennzeichnet, dass Komponente (b) eine aus 2-Hexylimidazol bestehende Stickstoffbase ist.

**19.** Protonen leiter nach Anspruch 1, dadurch gekennzeichnet, dass er aus einem Gemisch aus 3 Mol Imidazol und 1 Mol Imidazoliumtrifluoracetat besteht.

**20.** Protonenleiter nach Anspruch 1, dadurch gekennzeichnet, dass er aus einem Gemisch aus 4 Mol Imidazol und 1 Mol Imidazoliumbis(trifluormethansulfonimid) besteht.

**21.** Protonenleiter nach Anspruch 1, dadurch gekennzeichnet, dass er aus einem Gemisch aus 4 Mol Imidazol und 1 Mol Imidazoliumbisfluorsulfonimid besteht.

**22.** Protonenleiter nach Anspruch 1, dadurch gekennzeichnet, dass er aus einem Gemisch aus 3 oder 4 Mol 2-Hexylimidazol und 1 Mol 2-Hexylimidazolium besteht.

**23.** Flüssigelektrolyt, dadurch gekennzeichnet, dass er aus einem Protonenleiter nach einem der Ansprüche 1 bis 22 besteht.

**24.** Polymerelektrolyt, dadurch gekennzeichnet, dass er einen Protonenleiter nach einem der Ansprüche 1 bis 22, gelöst in einem Polymer, das zumindest eine polare Gruppe aufweist, umfasst.

**25.** Polymerelektrolyt nach Anspruch 24, dadurch gekennzeichnet, dass das Polymer Poly(ethylenoxid) ist.

**26.** Elektrochromes System, dadurch gekennzeichnet, dass es zwei transparente Halbleiter-Elektroden, die einander gegenüberliegend und voneinander beabstandet angeordnet sind, wobei jede der Elektroden auf einer Seite an einem transparenten Träger befestigt ist und auf der anderen Seite einen Überzug aus einem Halbleiter mit einer breiten verbotenen Zone aufweist, sowie einen Polymerelektrolyten nach einem der Ansprüche 24 oder 25 umfasst, der zwischen den Elektroden angeordnet ist und mit dem Halbleiter-Überzug in Kontakt steht.

**27.** Elektrochromes System, dadurch gekennzeichnet, dass es zwei transparente Halbleiter-Elektroden, die einander gegenüberliegend und voneinander beabstandet angeordnet sind, wobei jede der Elektroden auf einer Seiten an einem transparenten Träger befestigt ist, sowie einen Polymerelektrolyten nach einem der Ansprüche 24 oder 25 umfasst, der zwischen den Elektroden angeordnet ist und mit der anderen Seite jeder Elektrode in Kontakt steht, wobei dem Polymerelektrolyten zumindest ein Redoxfarbstoff zugesetzt ist.

**28.** Elektrochromes System, dadurch gekennzeichnet, dass es zwei transparente Halbleiter-Elektroden, die einander gegenüberliegend und voneinander beabstandet angeordnet sind, wobei jede der Elektroden auf einer Seite an einem transparenten Träger befestigt ist und eine der Elektroden auf der anderen Seite einen Überzug aus einem Halbleiter mit breiter verbotener Zone aufweist, sowie einen Polymerelektrolyten nach einem der Ansprüche 24 oder 25 umfasst, der zwischen den Elektroden angeordnet ist und mit dem Halbleiter-Überzug in Kontakt steht, wobei dem Polymerelektrolyten zumindest ein Redoxpaar zugesetzt ist, das zum Halbleiter mit breiter verbotener Zone komplementär ist.

**29.** Elektrochemische Zelle, bestehend aus einer Anode, einer Kathode und einem zwischen der Anode und der Kathode angeordneten Elektrolyten nach einem der Ansprüche 23 bis 25.

**30.** Elektrochemischer Hochleistungs-Kondensator, bestehend aus einer Anode, einer Kathode und einem zwischen der Anode und der Kathode angeordneten Elektrolyten nach einem der Ansprüche 23 bis 25.

**31.** Sensor, umfassend einen Protonenleiter nach einem der Ansprüche 1 bis 22.

**32.** Wasserfreies protisches Lösungsmittel, dadurch gekennzeichnet, dass es aus einem Protonenleiter nach einem der Ansprüche 1 bis 22 besteht.

**33.** Verwendung eines wasserfreien protischen Lösungsmittels nach Anspruch 32 zur Durchführung chemischer, photochemischer oder elektrochemischer Reaktionen.

## Claims

**1.** A proton conductor, characterized in that it is comprised of a mixture of the following components (a) and (b) :

a) an acid addition salt of a nitrogen base, having the formula :

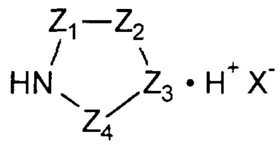

wherein :

$Z_1$, $Z_2$, $Z_3$ et $Z_4$, identical or different, each represent -N= or -C($Y_i$)= in which $Y_i$ represents a hydrogen atom, a linear or branched alkyl group having 1 to 20 carbon atoms, a fluoroalkyl radical having 1 to 20 carton atoms, or an oxoalkyl or azaalkyl radical having 1 to 20 carbon atoms, with the proviso that at least one and at most two of the groups $Z_1$, $Z_2$, $Z_3$ et $Z_4$ represent -N=, two adjacent carbon atoms being optionally hydrogenated and the nitrogen base optionally being part of a polymeric network, and

$X^-$ represents an anion derived from an acid selected from the group consisting of sulfonic acids of formula $R_FSO_3H$, sulfonimides of formula ($R_FSO_2$) ($R'_FSO_2$)NH and methides of formula ($R_FSO_2$) ($R'_FSO_2$)$CH_2$ or ($R_FSO_2$) ($R'_FSO_2$) ($R''_FSO_2$)CH, in which $R_F$, $R'_F$ or $R''_F$ each represent independently a radical $F(CF_2)_n$-, n being comprised between 0 and 6, the acid optionally being part of a polymeric network ; and

b) a nitrogen base having the formula :

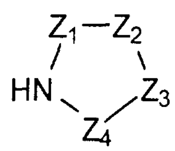

in which $Z_1$, $Z_2$, $Z_3$ et $Z_4$ have the aforesaid meanings, the nitrogen base optionally being part of a polymeric network ; and wherein components (a) and (b) are present in proportions to form a composition having a melting point lower than 25°C.

**2.** A proton conductor according to claim 1, characterized in that component (a) is an acid addition salt of a nitrogen base consisting of an imidazole having the formula :

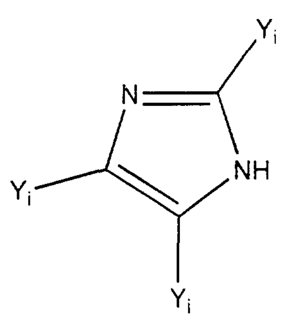

in which $Y_i$ has the aforesaid meaning.

**3.** A proton conductor according to claim 1, characterized in that component (a) is an acid addition salt of a nitrogen base consisting of a 1,2,3-triazole having the formula :

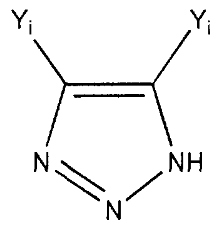

in which $Y_i$ has the aforesaid meaning.

**4.** A proton conductor according to claim 1, characterized in that component (a) is an acid addition salt of a nitrogen base consisting of 1,3,4-triazole having formula:

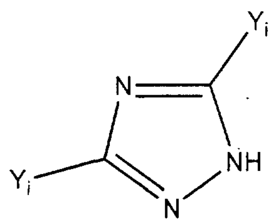

in which $Y_i$ has the aforesaid meaning.

**5.** A proton conductor according to claim 1, characterized in that component (a) is an acid addition salt of a nitrogen base consisting of a pyrazole having the formula :

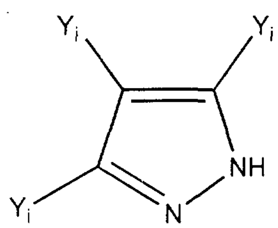

in which $Y_i$ has the aforesaid meaning.

**6.** A proton conductor according to claim 1, characterized in that component (a) is an acid addition salt of a nitrogen base consisting of an imidazoline having the following formula :

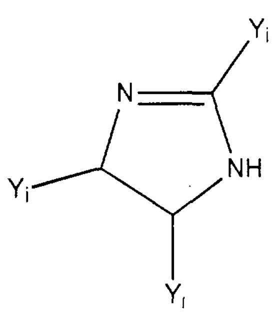

in which $Y_i$ has the aforesaid meaning.

**7.** A proton conductor according to anyone of claims 1 to 6, characterized in that the acid is selected from the group consisting of triflic acid, bisfluorosulfonimide, bistrifluoromethanesulfonimide, bistrifluoromethanesulfonylmethane, tristrifluoromethanesulfonylmethane and trisfluorosulfonylmethane.

**8.** A proton conductor according to claim 2, characterized in that component (a) is an addition salt of imidazole with triflic acid.

**9.** A proton conductor according to claim 2, characterized in that component (a) is an addition salt of imidazole with bisfluorosulfonimide.

**10.** A proton conductor according to claim 2, characterized in that component (a) is an addition salt of imidazole with bistrifluoromethanesulfonimide.

**11.** A proton conductor according to claim 2, characterized in that component (a) is an addition salt of 2-hexylimidazole with triflic acid.

**12.** A proton conductor according to any of claims 1 to 11, characterized in that component (b) is a nitrogen base consisting of an imidazole having the formula :

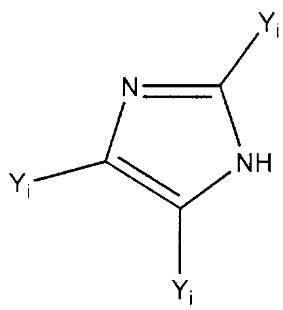

in which $Y_i$ has the aforesaid meaning.

**13.** A proton conductor according to any of claims 1 to 11, characterized in that component (b) is a nitrogen base consisting of 1,2,3-triazole having the formula :

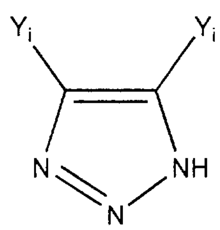

in which $Y_i$ has the aforesaid meaning.

**14.** A proton conductor according to any of claims 1 to 11, characterized in that component (b) is a nitrogen base consisting of a 1,3,4-triazole having formula :

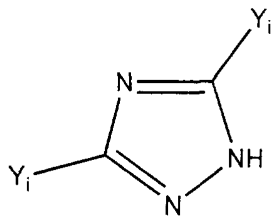

in which $Y_i$ has the aforesaid meaning.

**15.** A proton conductor according to any of claims 1 to 10, characterized in that component (b) is a nitrogen base consisting of a pyrazole having formula:

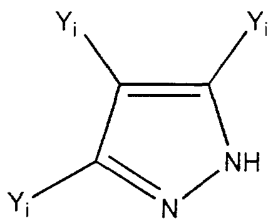

in which $Y_i$ has the aforesaid meaning.

**16.** A proton conductor according to any of claims 1 to 11, characterized in that component (b) is a nitrogen base consisting of an imidazoline having formula:

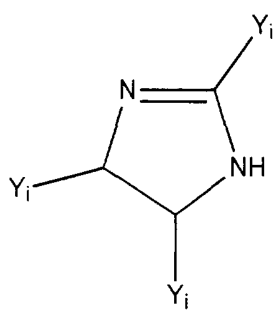

in which $Y_i$ has the aforesaid meaning.

**17.** A proton conductor according to any of claims 1 to 11, characterized in that component (b) is a nitrogen base consisting of imidazole.

**18.** A proton conductor according to any of claims 1 to 11, characterized in that component (b) is a nitrogen base consisting of 2-hexylimidazole.

**19.** A proton conductor according to claim 1, characterized in that it consists of a mixture of 3 moles of imidazole and one mole of imidazolium triflate.

**20.** A proton conductor according to claim 1, characterized in that it consists of a mixture of 4 moles of imidazole and one mole of imidazolium bis(trifluoromethanesulfonimide).

**21.** A proton conductor according to claim 1, characterized in that it consists of a mixture of 4 moles of imidazole and one mole of imidazolium bisfluorosulfonimide.

**22.** A proton conductor according to claim 1, characterized in that it consists of a mixture of 3 or 4 moles of 2-hexylimidazole and one mole de 2-hexylimidazolium.

**23.** A liquid electrolyte characterized in that it consists of a proton conductor as defined in any one of claims 1 to 22.

**24.** A polymer electrolyte characterized in that it comprises a proton conductor as defined in anyone of claims 1 to 22, dissolved in a polymer comprising at least one polar group.

**25.** A polymer electrolyte according to claim 24, characterized in that the polymer is polyethylene oxide.

**26.** An electrochrome system characterized in that it comprises two transparent semi-conducting electrodes arranged in space-apart opposed relationship to one another, each of said electrodes being fixed on one side thereof to a transparent support and comprising on an other side thereof a coating of a wide band gap semi-conducting material, and a polymer electrolyte as defined in claims 24 or 25, disposed between said electrodes and contacting the coatings of semi-conducting material.

**27.** An electrochrome system characterized in that it comprises two transparent semi-conducing electrodes arranged in spaced-apart opposed relationship to one another, each of said electrodes being fixed on one side thereof to a transparent support, and a polymer electrode as defined in claims 24 or 25, disposed between said electrodes and contacting the other side of each electrode, said polymer electrolyte comprising at least one redox coloring agent added thereto.

**28.** An electrochrome system characterized in that it comprises two transparent semi-conducting electrodes arranged in spaced-apart opposed relationship to one another, each of said electrodes being fixed on one side thereof to a transparent support and one of said electrodes comprising on an other side thereof a coating of a wide band gap semi-conducting material, and a polymer electrolyte as defined in claims 24 or 25, disposed between said electrodes and contacting the coating of semi-conducting material, said polymer electrolyte comprising at least one redox couple added thereto, said redox couple being complementary to said wide band gap semi-conducting material.

**29.** An electrochemical generator comprising an anode, a cathode and an electrolyte as defined in anyone of claims 23 to 25, disposed between the anode and the cathode.

**30.** An electrochemical supercapacitor comprising an anode, a cathode and an electrolyte as defined in anyone of claims 23 to 25, disposed between the anode and the cathode.

**31.** A sensor comprising a proton conductor as defined in anyone of claims 1 to 22.

**32.** An anhydrous protic solvent consisting of a proton conductor as defined in anyone of claims 1 to 22.

**33.** Use of an anhydrous protic solvent as defined in claim 32, for carrying out chemical, photochemical or electrochemical reactions.

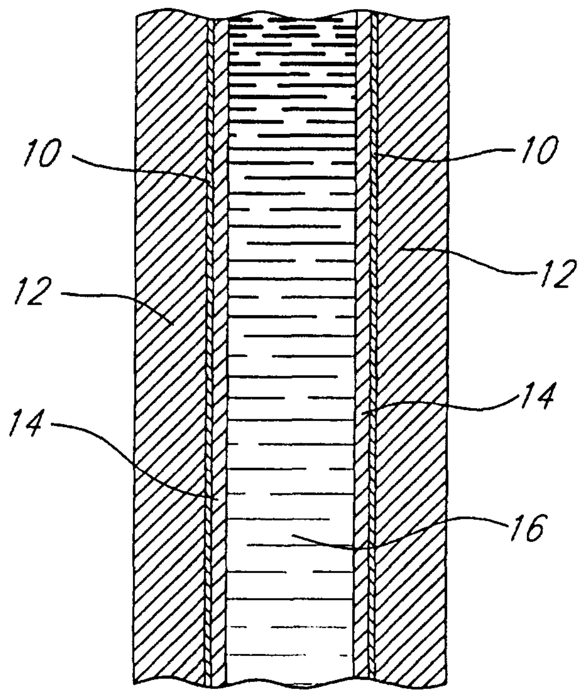


FIG. 1

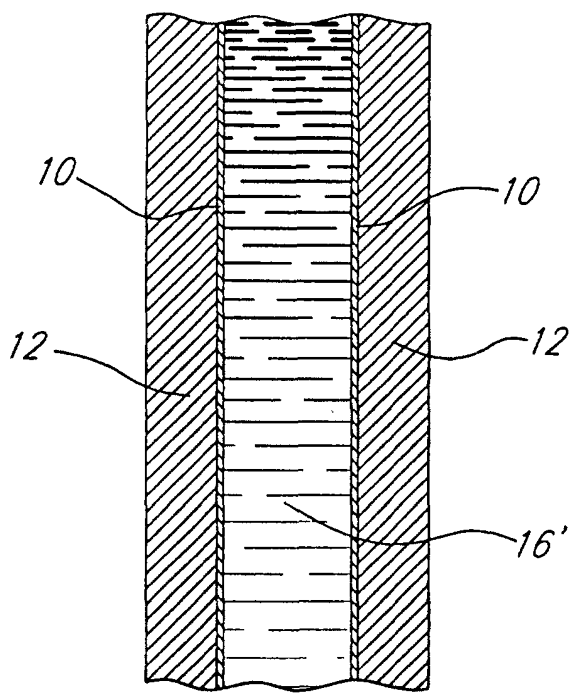


FIG. 2

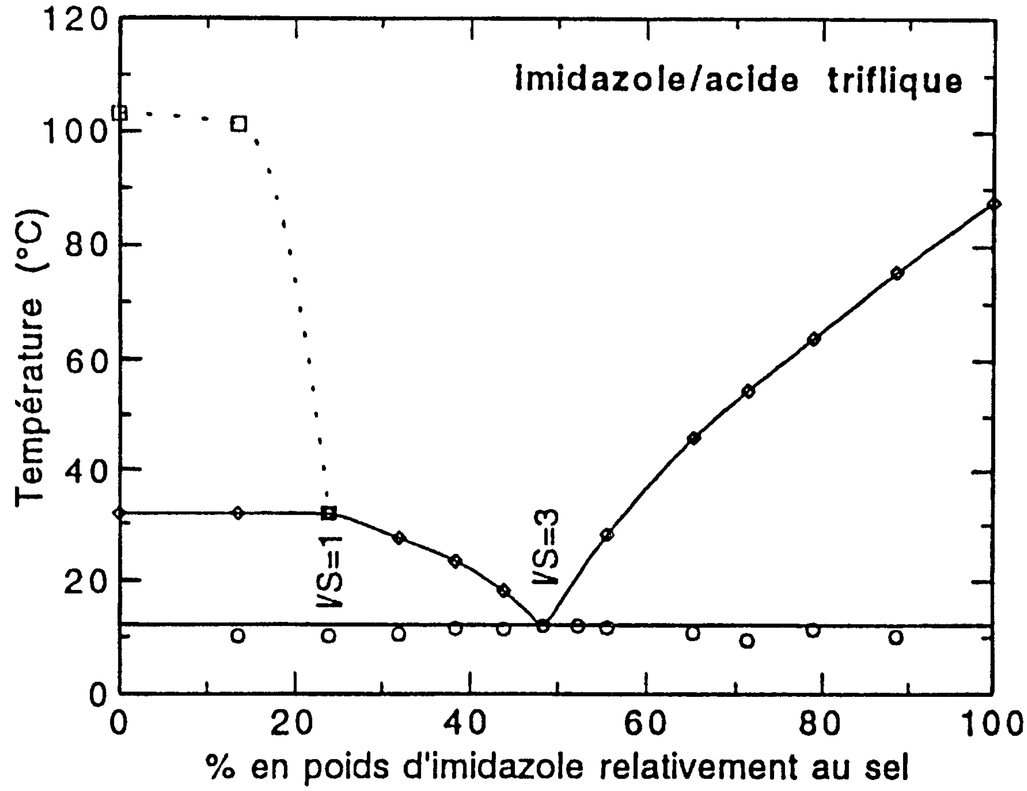
imidazole/acide triflique
Température (°C)
% en poids d'imidazole relativement au sel
I/S=1
I/S=3
120
100
80
60
40
20
0
0
20
40
60
80
100

FIG. 3

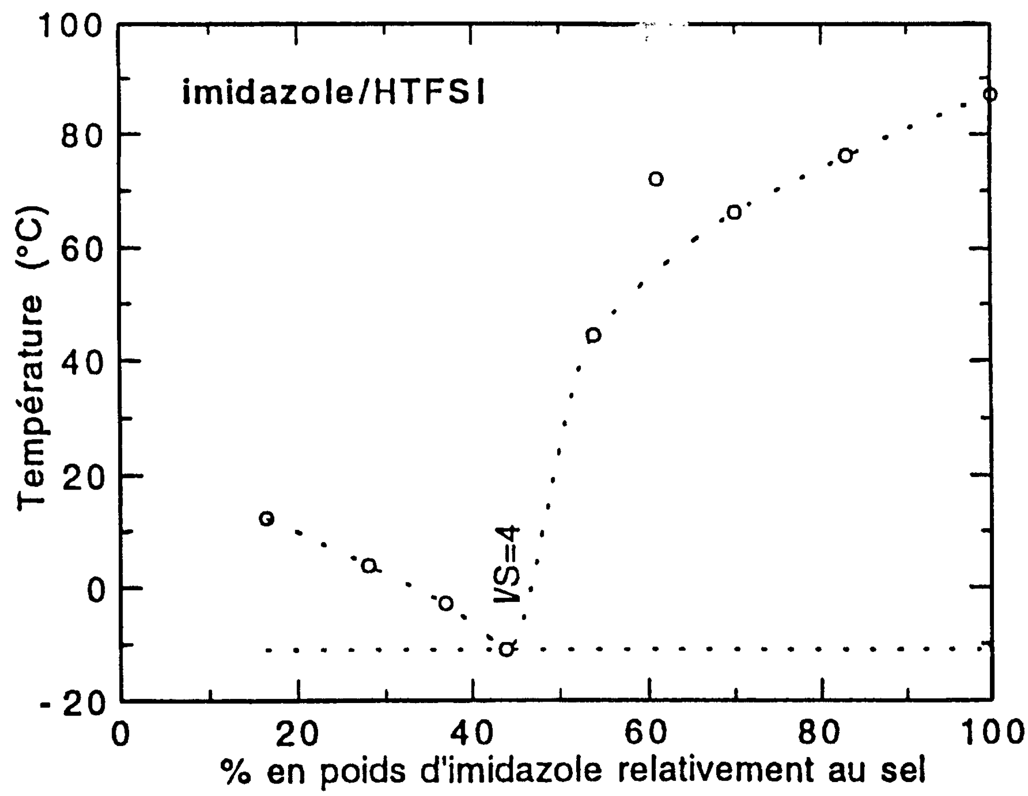
imidazole/HTFSI
Température (°C)
% en poids d'imidazole relativement au sel
I/S=4
100
80
60
40
20
0
-20
0
20
40
60
80
100

FIG. 4

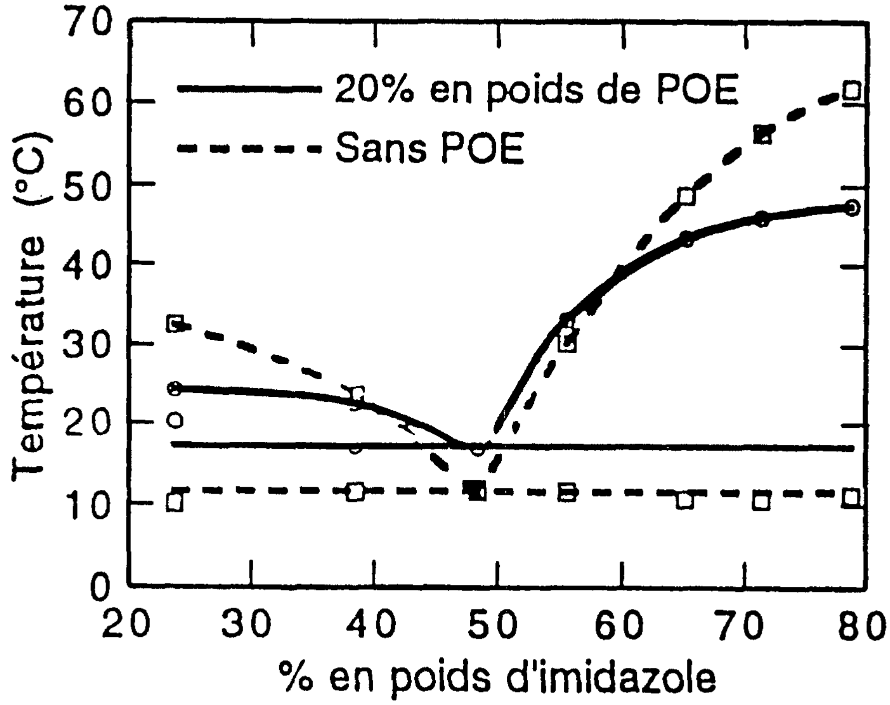

70
60
50
40
30
20
10
0
20
30
40
50
60
70
80
20% en poids de POE
Sans POE
Température (°C)
% en poids d'imidazole


FIG. 5

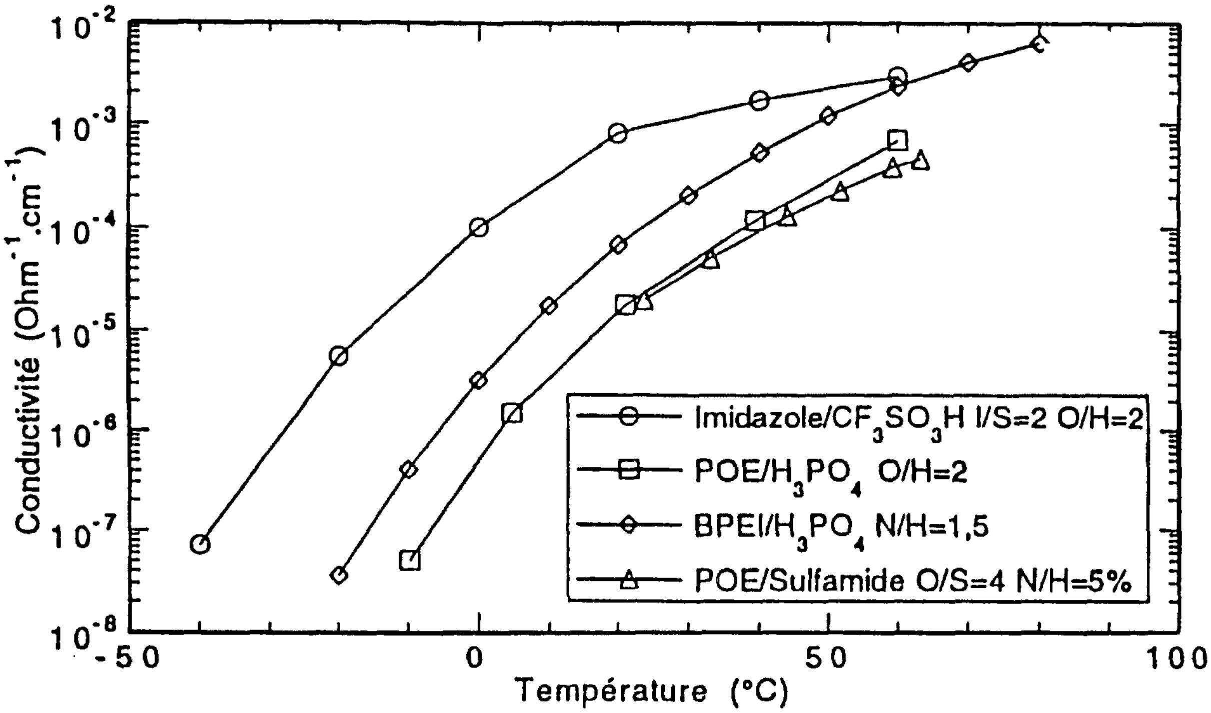

$10^{-2}$
$10^{-3}$
$10^{-4}$
$10^{-5}$
$10^{-6}$
$10^{-7}$
$10^{-8}$
-50
0
50
100
Conductivité (Ohm$^{-1}$.cm$^{-1}$)
Température (°C)
Imidazole/$CF_3SO_3H$ I/S=2 O/H=2
POE/$H_3PO_4$ O/H=2
BPEI/$H_3PO_4$ N/H=1,5
POE/Sulfamide O/S=4 N/H=5%


FIG. 6

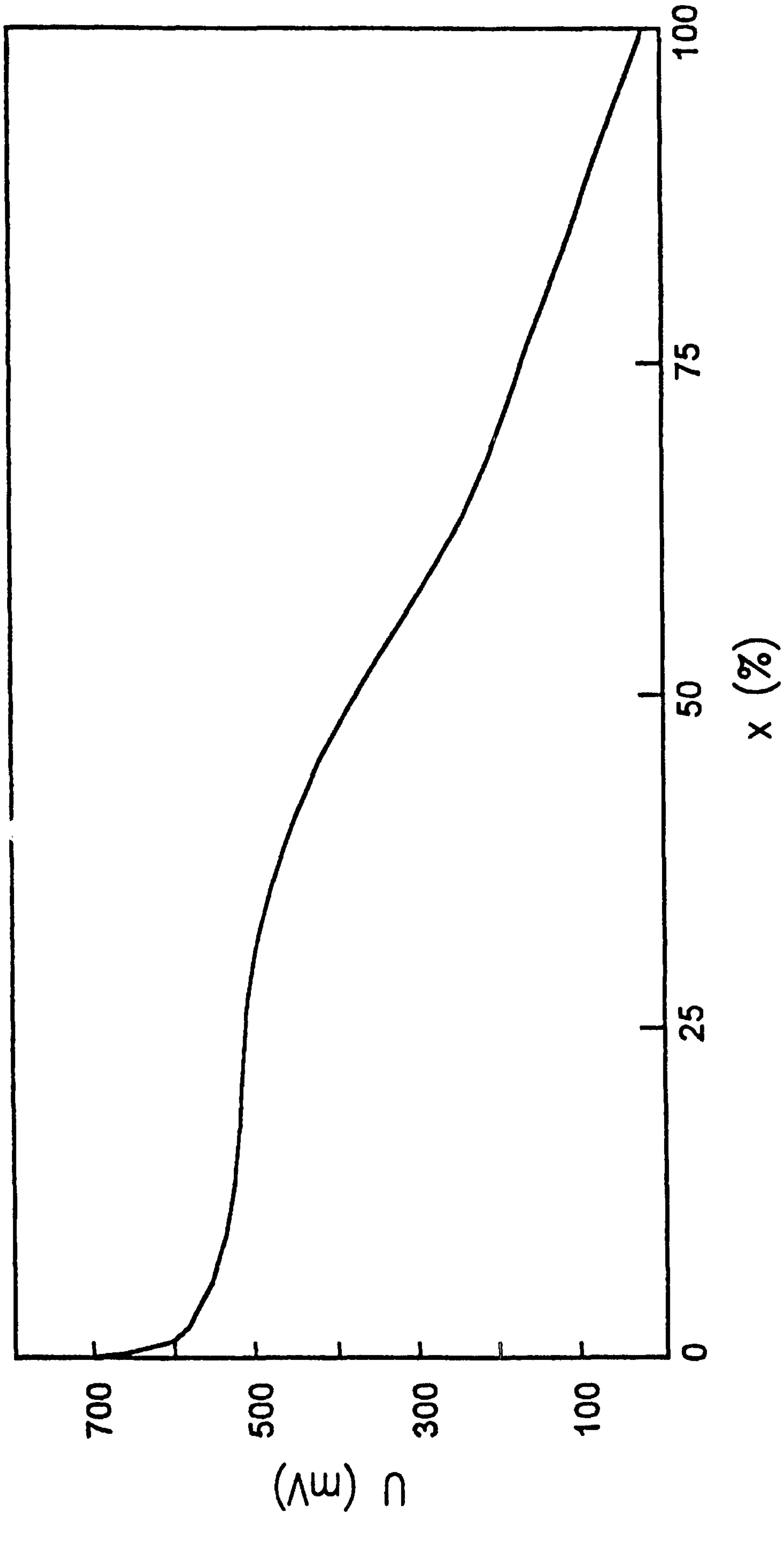


FIG_7

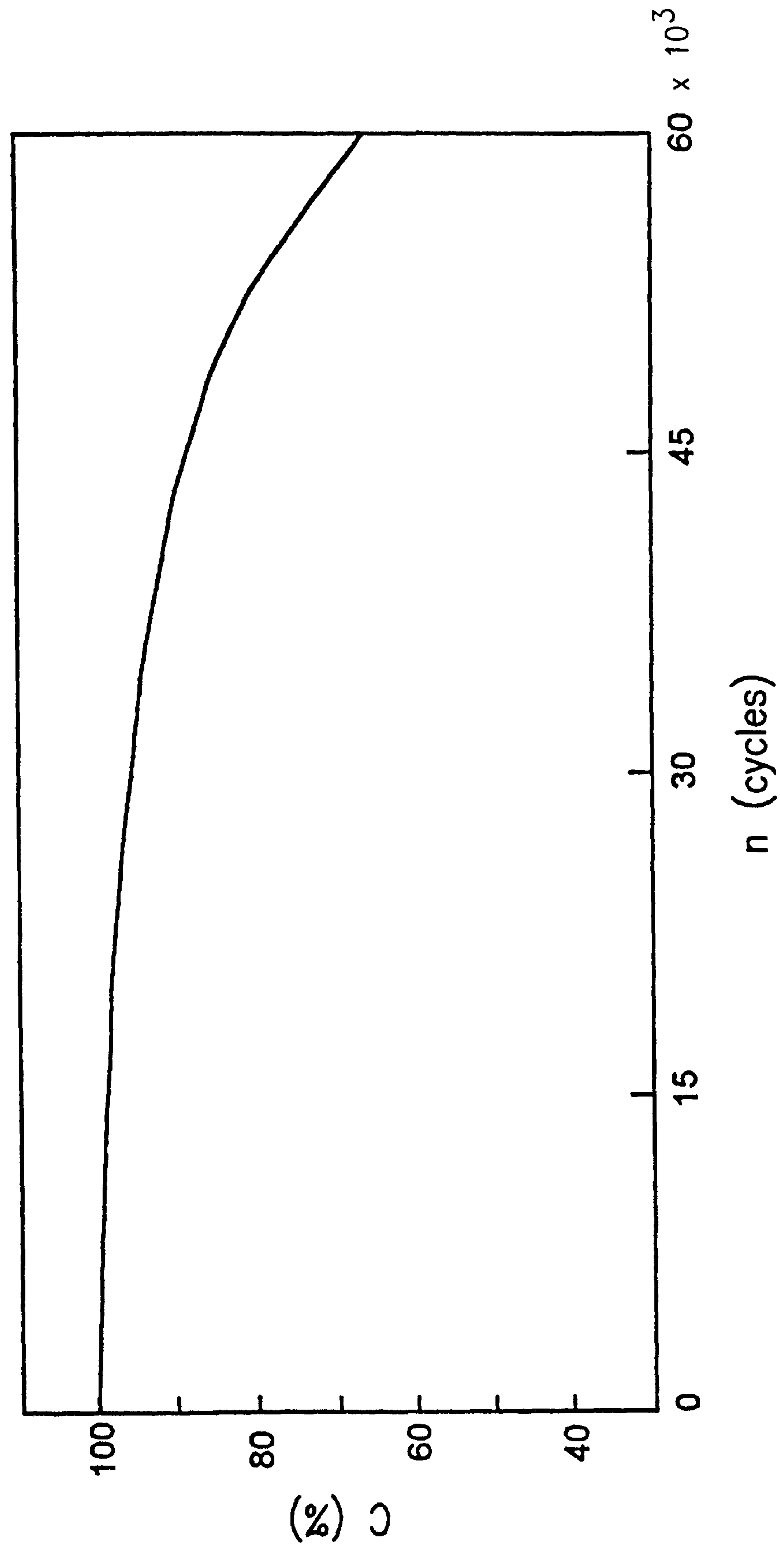


FIG_8